# EUROPEAN PATENT APPLICATION

(11) **EP 1 747 751 A2**
(43) Date of publication of application: **31.01.2007**
(21) Application number: 06015810.2
(22) Date of filing: 28.07.2006
(51) Int. Cl.: A61B 5/00, A61B 18/24

(54) **Optical diagnosis and treatment apparatus**

(30) Priority: 29.07.2005 JP 2005220064
(71) Applicant: Fujinon Corporation, Kita-ku Saitama-shi Saitama-ken (JP)
(72) Inventor: Kohno, Shinichi, Kita-ku Saitama-shi Saitama-ken (JP)
(74) Representative: Klunker . Schmitt-Nilson . Hirsch

(57) **Abstract**

In an optical diagnosis and treatment apparatus including a pulsed light source (10), an illumination optical system (15) for illuminating a region (11) of a living body through a guide tube (12), a light condensing means (16), an optical scan means (17), a light detection means (24) for detecting the pulsed light (14) reflected from the region (11), an operation means (29) for reconstructing, based on an output from the light detection means (24), a tomographic image of the region (11), an image display means (30) for displaying the image based on an output from the operation means (29) and a light intensity switching means (25, 51) for switching the intensity of the pulsed light (14) at least between a level at which vaporization of living body tissue due to multi-photon absorption occurs at a convergence position of the light (14) and a level at which vaporization does not occur.

## Description

### BACKGROUND OF THE INVENTION

### Field of the Invention

The present invention relates to an optical diagnosis and treatment apparatus for noninvasively diagnosing the condition of an internal region of a living body and for noninvasively treating a disease in the region.

### Description of the Related Art

Conventionally, various kinds of diagnostic apparatuses which can display a tomographic image of an internal region of a living body have been provided for practical use, for example, to judge the degree of invasiveness of a cancer. Among such diagnostic apparatuses, apparatuses which can diagnose patients without celiotomy and thoracotomy are noninvasive diagnostic apparatuses. Since the apparatuses have an advantage that burdens on other regions of the living bodies of the patients can be reduced, many kinds of apparatuses have been studied and developed recently.

As examples of the diagnostic apparatuses, as described above, an ultrasound diagnostic apparatus, an optical coherence tomographic diagnostic apparatus and the like are well known. However, in the ultrasound diagnostic apparatus, it is necessary that water intervenes between an ultrasonic vibrator and a living body. Therefore, there are problems that a complex technique is required and that a frame rate becomes extremely slow because of a physical limit imposed by the sound speed. Further, in the optical coherence tomographic diagnostic apparatus, the structure of an optical system is complex and precise. Therefore, there are problems that it is difficult to reduce the size of the apparatus and that the production cost is high.

Under these circumstances, apparatuses which can display tomographic images of internal regions of living bodies using pulsed light have been proposed, as disclosed in U.S. Patent No. 5, 305, 759. The apparatuses are structured, for example, as endoscopes. In such apparatuses, a region of a living body is illuminated with pulsed light through a guide tube of an endoscope and reflected light of the pulsed light is detected. Then, information about the region of the living body with respect to the depth direction of the living body, in other words, with respect to the illumination direction of the pulsed light is obtained based on the detection time of the reflected light. The information about the region of the living body is obtained by utilizing the characteristic of the reflected light that it returns at different time based on a reflection position with respect to the depth direction of the relevant region. The reflection position is a position on a boundary plane between two composition elements of the living body, which have different refractive indices from each other. Then, a tomographic image is reconstructed based on the information and displayed.

The optical diagnostic apparatuses disclosed in U.S. Patent No. 5,305,759 can noninvasively display tomographic images of an internal region of a living body. However, in the apparatuses, even if cancer tissue or the like is detected in a region of the living body, it is impossible to treat the cancer. Therefore, it is necessary to use a separate apparatus to treat the cancer. Conventionally, for example, an endoscopic apparatus which can treat a disease through a treatment tool insertion channel of an endoscope is well known. In the endoscopic apparatus, a diseased region is removed with mechanical forceps or cauterized using high frequency current. If the optical diagnostic apparatuses disclosed in U.S. Patent No. 5,305,759 are compared with the endoscopic apparatus, as described above, the operation efficiency of the optical diagnostic apparatuses in diagnosis and treatment is not so good as that of the endoscopic apparatus.

### SUMMARY OF THE INVENTION

In view of the foregoing circumstances, it is an object of the present invention to provide an optical diagnosis and treatment apparatus which can noninvasively display a tomographic image for diagnosis, and which can also treat a diseased region of a living body.

An optical diagnosis and treatment apparatus according to the present invention is an apparatus which can diagnose a patient by utilizing pulsed light to reconstruct a tomographic image. Specifically, the optical diagnosis and treatment apparatus is an optical diagnosis and treatment apparatus comprising:
a pulsed light source for emitting pulsed light;
a guide tube which is inserted into a living body;
an illumination optical system for illuminating a region of the living body with the pulsed light through the guide tube;
a light condensing means for condensing the pulsed light emitted from the illumination optical system;
an optical scan means for two-dimensionally scanning the region of the living body with the condensed pulsed light;
a light detection means for detecting the pulsed light reflected from the region;
an operation means for reconstructing, based on an output from the light detection means, a tomographic image of the region which has been illuminated with the pulsed light;
an image display means for displaying the tomographic image based on an output from the operation means; and
a light intensity switching means for switching the intensity of the pulsed light, with which the region is illuminated, at least between two levels, wherein the two levels are a level at which vaporization of living body tissue due to multi-photon absorption occurs at a convergence position of the pulsed light by the light condensing means and a level at which vaporization does not occur.

It is particularly preferable that a so-called femtosecond laser is used as the pulsed light source. The femtosecond laser emits pulsed light which has an fs-order (femtosecond-order) pulse width.

Meanwhile, the light intensity switching means may be a means for changing an output from the pulsed light source, for example. Alternatively, the light intensity switching means may be an ND (Neutral Density) filter or the like. The ND filter is a filter which is insertable into and removable from the optical path of the pulsed light emitted from the pulsed light source. When the ND filter is inserted into the optical path, it attenuates the pulsed light.

Further, it is preferable that the light condensing means includes a variable focus mechanism which can change the convergence position of the pulsed light.

Further, it is preferable that the optical diagnosis and treatment apparatus according to the present invention, which is structured as described above, is an apparatus further comprising:
a control means for setting the convergence position of the pulsed light with respect to the direction of the two-dimensional scanning and/or the depth direction of illumination thereof based on position information about the reconstructed tomographic image when the intensity of the pulsed light is set at the level at which the vaporization occurs.

The optical diagnosis and treatment apparatus according to the present invention includes the light intensity switching means for switching the intensity of the pulsed light, with which a region of a living body is illuminated, at least between two levels, namely a level at which vaporization of living body tissue due to multi-photon absorption occurs at a convergence position of the pulsed light by the light condensing means and a level at which vaporization does not occur. Therefore, it is possible to reconstruct and display a tomographic image by illuminating an internal region of a living body with pulsed light at the level at which vaporization does not occur. Further, it is possible to treat a cancer or the like by removing cancer tissue, for example.

If a femtosecond laser is used as the pulsed light source, it becomes possible to utilize fs-order pulsed light, which has a very short pulse width. Therefore, when light reflected from the living body is temporally resolved and detected, high temporal resolution light detection is achieved. Hence, it becomes possible to reconstruct an extremely precise tomographic image. Further, the intensity of the pulsed light which has a short pulse width, as described above, can be very high. Therefore, if such pulsed light is utilized, it is possible to efficiently vaporize living body tissue.

Meanwhile, if the condensing means includes a variable focus mechanism which can change the convergence position of the pulsed light, it is possible to easily control the depth of living body tissue which is vaporized. The depth can be controlled by appropriately changing the convergence position of the pulsed light so that the depth corresponds to the invasion condition of cancer tissue.

Further, if the optical diagnosis and treatment apparatus according to the present invention includes the control means for setting the convergence position of the pulsed light with respect to the direction of two-dimensional scanning and/or the depth direction of illumination thereof based on position information about the reconstructed tomographic image when the intensity of the pulsed light is set at the level at which the vaporization occurs, it is possible to accurately set the convergence position of the pulsed light at an appropriate position with reference to a displayed tomographic image. The convergence position of the pulsed light is a position at which vaporization occurs.

### BRIEF DESCRIPTION OF THE DRAWINGS

Figure 1 is a schematic diagram illustrating a side view of an optical diagnosis and treatment apparatus according to an embodiment of the present invention;
Figure 2 is a schematic diagram illustrating an example of tomographic images displayed in the optical diagnosis and treatment apparatus in Figure 1;
Figure 3A is a diagram for explaining a scanning state and an illumination state of pulsed laser light in the optical diagnosis and treatment apparatus illustrated in Figure 1;
Figure 3B is a diagram for explaining a scanning state and an illumination state of pulsed laser light in the optical diagnosis and treatment apparatus illustrated in Figure 1; and
Figure 4 is a perspective view illustrating an example of a light intensity switching means which is used in the optical diagnosis and treatment apparatus according to the present invention.

### DESCRIPTION OF THE PREFERRED EMBODIMENTS

Hereinafter, an embodiment of the present invention will be described in detail with reference to the attached drawings.

An optical diagnosis and treatment apparatus in an embodiment of the present invention is an apparatus, of which a part is incorporated into an endoscope, for example. The optical diagnosis and treatment apparatus includes a femtosecond laser (hereinafter, referred to as an fs laser) 10, a guide tube 12, an illumination optical system 15, a condensing lens 16 and an optical scan means 17. The fs laser 10 is a pulsed light source which emits pulsed laser light. The guide tube 12 is provided as an element of the endoscope, and the leading edge of the guide tube 12 is inserted into the inside of a living body 100, such as a human body. The illumination optical system 15 illuminates or irradiates a region (for example, the surface of a mucous membrane) 11 in the living body, through the guide tube 12, with the pulsed laser light 14 which is emitted from the fs laser 10. The condensing lens 16 condenses the pulsed laser light 15 emitted from the illumination optical system 15. The optical scan means 17 two-dimensionally scans the region 11 with the condensed pulsed light 14.

The illumination optical system 15 includes an optical fiber 20, an optical fiber 21 and a fiber coupler 22. The optical fiber 20 is optically connected to a light emitting portion of the fs laser 10. The leading edge of the optical fiber 21 is housed in the guide tube 12. The fiber coupler 22 couples the optical fibers 21 and 22. Here, the optical fiber 21, the condensing lens 16 and the optical scan means 17 may be integrated to form a probe, which is inserted into a forceps insertion hole (not illustrated) in the guide tube 12. In that case, the diameter of the probe should be approximately 10mm.

The condensing lens 16 is a so-called fluid focus lens. The condensing lens 16 includes a fluid lens 16a and a drive unit 16b. The fluid lens 16a is made of two kinds of fluid which do not mix together, and which form an interface therebetween. The drive unit 16b changes the shape of the interface by changing the surface tension of the fluid by application of a direct current voltage to the fluid. If the condensing lens 16 is structured, as described above, the fluid lens 16a can be formed into a convex lens in certain voltage application condition. Further, if the voltage applied to the fluid lens 16a is changed, the shape of the interface is changed, and the focal length of the fluid lens 16a can be changed.

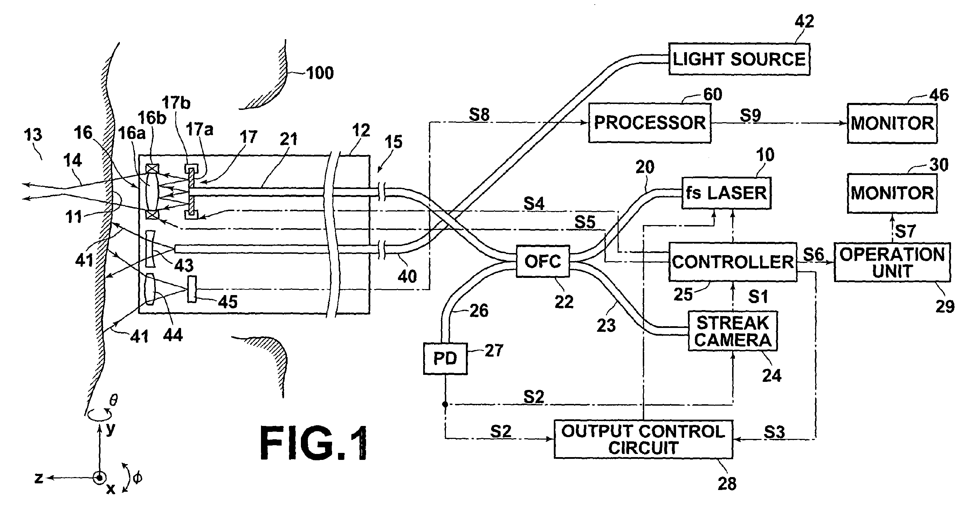

Meanwhile, the optical scan means 17 is an MEMS (Micro Electro Mechanical Systems) device which is monolithically formed of single-crystal silicon material, for example. The optical scan means 17 includes a micromirror 17a and a drive unit 17b. The drive unit 17b swings the micromirror 17a so that the micromirror 17a rotates about each of two axes. In this example, both of the two axes are perpendicular to the axial direction of the optical fiber 21. The two axes are an x-axis, which extends in a direction perpendicular to the plane on which Figure 1 is drawn, and a y-axis, which extends in a vertical direction in Figure 1. The micromirror 17a swings in a direction φ with respect to the x-axis. The micromirror 17a swings in a direction θ with respect to the y-axis. Here, a mechanism which drives the micromirror 17a by electromagnetic force, a mechanism which drives the micromirror 17a by electrostatic force, or the like may be appropriately adopted as the drive unit 17b.

Further, the optical diagnosis and treatment apparatus includes an optical fiber 23, a streak camera 24, a controller 25, an optical fiber 26 and a photodetector 27, such as a photodiode, for example. The optical fiber 23 is connected to the fiber coupler 22 so that the pulsed laser light 14, which has been reflected from internal tissue 13 of the region 11, and which has returned through the optical fiber 21, enters the optical fiber 23. The streak camera 24 is optically connected to the optical fiber 23 and detects the pulsed laser light 14 which has returned. The controller 25 receives an output S1 from the streak camera 24. The optical fiber 26 is connected to the fiber coupler 22 so that a part of the pulsed laser light 14, which has propagated through the optical fiber 20, is branched to enter the optical fiber 26. The photodetector 27 is connected to the optical fiber 26 to detect the pulsed laser light 14. The photodetector 27 is a photodiode, for example.

The streak camera 24 temporally resolves the pulsed laser light 14, which has been reflected from the internal tissue 13 of the region 11, and which has returned through the optical fiber 21, at resolution of fs-order (femtosecond-order), which is ultra-fast resolution, and detects the pulsed laser light 14. An output S1 (detection result) from the streak camera 24 is input to the controller 25.

The photodetector 27 is connected to an output control circuit 28 which controls an output from the fs laser 10. The output control circuit 28 is connected to the fs laser 10 and the controller 25. The controller 25 is connected to an operation apparatus 29, which reconstructs a three-dimensional tomographic image of the internal tissue 13, as will be described later. The operation apparatus 29 is connected to a monitor (image display means) 30, which displays the three-dimensional tomographic image.

Further, the controller 25 inputs an optical scan control signal S4 for controlling drive of the optical scan means 17 to the drive unit 17b. The controller 25 also inputs a focus control signal S5 for controlling the focal length of the condensing lens 16 to the drive unit 16b.

In addition to the elements, as described above, the optical diagnosis and treatment apparatus includes elements corresponding to those of a general endoscope. The elements corresponding to those of the endoscope will be described. The leading edge of a light guide 40 is housed in the guide tube 12 and the other end of the light guide 40 is connected to a light source 42, such as a white light source, for example. The light source 42 emits illumination light 41 for illuminating the region 11. Further, a lens 43 for spreading the illumination light 41 which is emitted from the light guide 40 is arranged in the guide tube 12. Further, an image formation lens 44 and an imaging means 45, such as a CCD (Charge Coupled Device), are arranged in the guide tube 12. The image formation lens 44 forms an image with the illumination light 41 reflected from the region 11, and the imaging means 45 captures an image of the surface of the region 11, which is formed by the image formation lens 44. Further, a processor 60 and a monitor (image display means) 46 are provided outside the guide tube 12. The processor 60 is electrically connected to the imaging means 45.

The operation of the optical diagnosis and treatment apparatus, which is structured as described above, will be described. First, an operation for diagnosis, in other words, an operation for reconstructing and displaying a tomographic image for diagnosis, will be described. In the operation for diagnosis, the light source 42 is turned on and illumination light 41 is emitted from the light source 42. The illumination light 41 propagates through the light guide 40, and the illumination light 41 is emitted from the leading edge of the guide tube 12. Accordingly, the region 11 is illuminated with the illumination light 41. The illumination light 41 is reflected from the surface of the region 11, and an image of the surface of the region 11 is formed by the image formation lens 44 with the reflected illumination light 41. Then, the image is captured by the imaging means 45, and an image signal S8 is output from the imaging means 45. The image signal S8 is input to the processor 60. The processor 60 processes the image signal S8, and outputs a video signal S9 to the monitor 46. Then, the monitor 46 displays an image of the surface of the region 11 based on the video signal S9.

Therefore, users of the apparatus, such as a surgeon, can observe an image displayed on the monitor 46 and determine a region, of which the tomographic image should be produced, based on the displayed image. Specifically, the users can determine a region which should be two-dimensionally scanned with the pulsed laser light 14 and a region on which treatment, as will be described later, should be performed.

In the present embodiment, an image of the region 11 is captured and displayed on the monitor 46 during diagnosis and treatment. Therefore, the users can observe the image displayed on the monitor 46 and check the illumination condition of the pulsed laser light 14. However, some kinds of imaging means 45, such as a CCD, may be affected by the pulsed laser light 14 which is reflected from the surface of the region 11 or the like. Such adverse effects can be prevented by providing an optical filter or the like between the image formation lens 44 and the imaging means 45, for example. The optical filter removes light, of which the wavelength is in the range of that of the pulsed laser light 14.

Meanwhile, an fs laser which emits pulsed laser light 14, of which the central wavelength is 800nm, is used as the fs laser 10. The wavelength of 800nm is a wavelength at which an absorption rate of light is the lowest among the wavelengths of 700nm through 1100nm, at which loss of light due to absorption by a living body is low. Further, the controller 25 also functions as a light intensity switching means for switching the output from the fs laser 10 between two levels, namely high output and low output. In this case, the high output is output at a level in which the intensity is sufficiently high to induce vaporization of living body tissue due to multi-photon absorption at a laser light convergence position (beam waist) by the condensing lens 16. The low output is output at a level in which vaporization at the laser light convergence position is not induced.

When diagnosis is performed, in other words, when a tomographic image of the internal tissue 13 of the region 11 is formed, the output from the fs laser is set at the low level. At this time, the pulsed laser light 14 which is emitted from the optical fiber 21 in a scattered light state is reflected from a sub-mirror (not illustrated), which is formed on the surface of the fluid lens 16a, toward the micromirror 17a. Then, the light is reflected from the micromirror 17a and transmitted through the fluid lens 16a. The light is condensed so as to converge in the internal tissue 13. The pulsed laser light 14 is reflected at the internal tissue 13 and transmitted through the fluid lens 16a and the micromirror 17a. Then, the pulsed laser light 14 enters the optical fiber 21 again.

The pulsed laser light 14 propagates through the optical fiber 21, the fiber coupler 22 and the optical fiber 23. Then, the pulsed laser light 14 is detected by the streak camera 24. When the region 11 is illuminated with a single pulse of pulsed laser light 14, the pulsed laser light 14 is reflected from the internal tissue 13, and the pulsed laser light 14 returns at different time based on a light reflection position with respect to the depth direction of the internal tissue 13. The light reflection position is a position on a boundary plane between two composition elements of the living body, which have different refractive indices from each other. The streak camera 24 temporally resolves the pulsed laser light 14, which is incidents thereon at different time, at resolution of fs-order (femtosecond-order), which is ultra-fast resolution, and detects the pulsed laser light 14. An output S1 (detection result) from the streak camera 24 is input to the controller 25. Detection time of the pulsed laser light 14 by the streak camera 24 corresponds to a light reflection position, and the intensity of the pulsed laser light 14 corresponds to the tissue condition of the living body, such as a light absorption characteristic, at each light reflection position. The detection time is converted into position information on a phosphor plane of the streak camera 24. Therefore, the output S1 represents information with respect to the depth direction of the internal tissue 13, in other words, information with respect to the z-axis in Figure 1.

The region 11 is sequentially illuminated by two-dimensionally scanning the region 11 with the pulsed laser light 14 using the optical scan means 17. Therefore, the output S1 (detection result) from the streak camera 24, which is sequentially input to the controller 25, represents information about the internal tissue 13 with respect to the depth direction at each scan position in two-dimensional scanning, as described above.

The controller 25 inputs the output S1 (detection result) and a scan position signal corresponding to the optical scan control signal S4 to the operation apparatus 29. The controller 25 inputs the output S1 and the scan position signal as reconstruction data S6 for reconstructing a three-dimensional tomographic image. The operation apparatus 29 reconstructs, based on the reconstruction data S6, a tomographic image of the region 11 in a predetermined two-dimensionally scanned region. Then, the operation apparatus 29 inputs image data S7, which represents the reconstructed image, to the monitor 30. The tomographic image is displayed on the monitor 30.

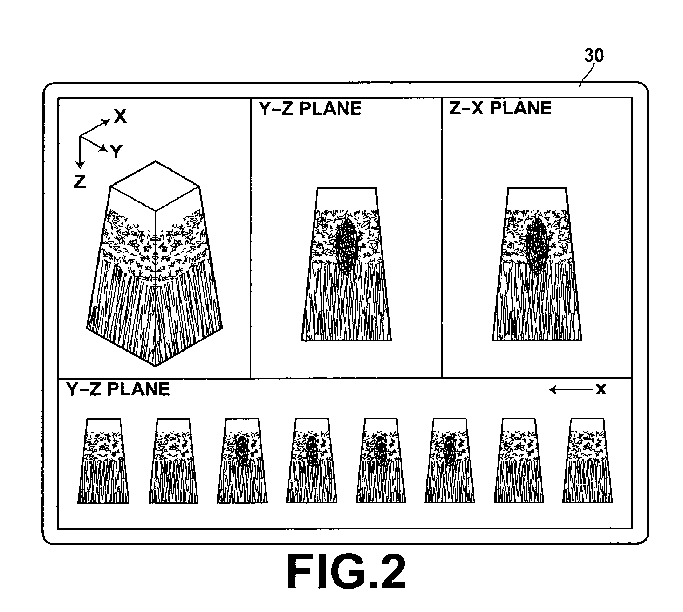

Figure 2 is a diagram illustrating an example of images displayed on the monitor 30. As illustrated in Figure 2, a quasi-three-dimensional image, a single tomographic image in a y-z plane and a single tomographic image in a z-x plane are sequentially displayed from the left side of the upper row. Further, a plurality of tomographic images in y-z planes is displayed in the lower row. The plurality of tomographic images in y-z planes is a plurality of tomographic images, of which the positions with respect to the direction of the x-axis are different from each other.

As illustrated in Figure 1, a part of the pulsed laser light 14 emitted from the fs laser 10 propagates through the fiber coupler 22 and the optical fiber 26. Then, the pulsed laser light 14 is detected by the photodetector 27. The photodetector 27 outputs a photodetection signal S2, and the photodetection signal S2 is input to the streak camera 24. The photodetection signal S2 is used as a trigger signal for starting electron sweep by the streak camera 24 in synchronization with emission of the pulsed laser light 14.

The photodetection signal S2, which is output by the photodetector 27, is also input to the output control circuit 28. The output control circuit 28 compares the photodetection signal S2 with an output setting signal S3, which is input from the controller 25. The output control circuit 28 accurately sets the output from the fs laser 10 at a predetermined value represented by the output setting signal S3 by changing the output from the fs laser 10 based on the comparison result.

Next, an operation for treatment of the living body 11 by the optical diagnosis and treatment apparatus in the present embodiment will be described. When treatment is performed, the controller 25 changes the setting of the fs laser 10 to high output, as described above. Then, the intensity of the pulsed laser light 14, with which the internal tissue 13 is illuminated, becomes a value which is sufficiently large to induce vaporization of the living body tissue due to multi-photon absorption at a convergence position (beam waist) of the pulsed laser light 14. Therefore, it is possible to perform treatment, such as removal of cancer cells which are present in the internal tissue 13, for example. If both diagnosis (displaying a tomographic image) and treatment can be performed using a single optical diagnosis and treatment apparatus, as described above, the operation efficiency of the apparatus in diagnosis and treatment is sufficiently high.

Meanwhile, if the pulse width of the pulsed laser light, with which the internal tissue 13 is illuminated, is on the order of nanoseconds, dielectric breakdown occurs and plasma is generated. Therefore, a shock wave and heat are generated, and there is a possibility that a region in the vicinity of the position which is illuminated with the pulsed laser light is also adversely affected. If the pulse width of the pulsed laser light is reduced to the order of picoseconds, plasma is not generated. Therefore, it is possible to prevent thermal denaturation or the like. If the pulse width of the pulsed laser light is further reduced to the order of femtoseconds, another physical process, namely, multiphoton absorption, such as two-photon absorption, is induced.

If the multiphoton absorption is utilized, it is possible to process a region, of which the size is less than the beam diameter of the pulsed laser light, with which the region is illuminated. Further, it becomes possible to process or treat an internal region of transparent substance. The internal region can be processed due to the unique characteristic of the pulsed laser light 14 that multiphoton absorption occurs only at a region in which the intensity of the pulsed laser light 14 is high.

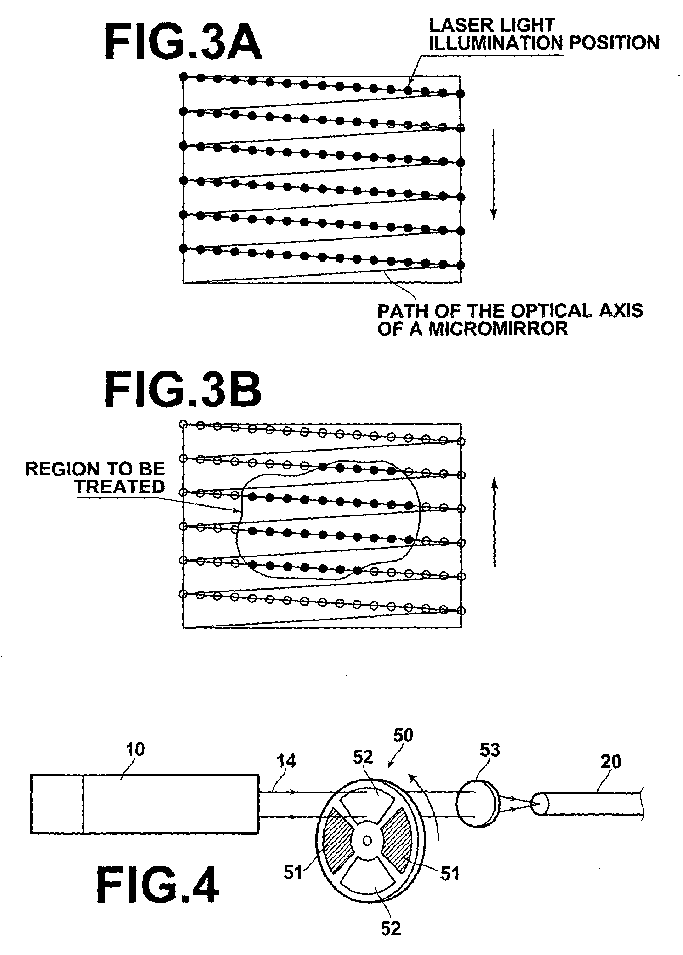

Next, execution timing of diagnosis and treatment, as described above, will be explained in detail with reference to Figures 3A and 3B. In Figures 3A and 3B, rectangles are two-dimensionally scanned regions. Further, a zigzag pattern in each of the rectangles represents the scan path of the optical axis of light reflected by a micromirror 17a. Further, black dots on the scan path represent the positions of spots which have been illuminated with the pulsed laser light 14.

Figure 3A illustrates a state during diagnosis. In this state, the scanning path moves from the top to the bottom in Figure 3A, and the pulsed laser light 14 is always emitted at predetermined time intervals. In the present embodiment, when a single vertical scan period of the pulsed laser light 14 ends, the next vertical scan is performed in the opposite direction, as illustrated in Figure 3B. While the region is scanned in the opposite direction, treatment is performed on the region. Specifically, when the region is vertically scanned in the opposite direction, the output from the fs laser 10 is switched to high output, as described above. When the region is vertically scanned in the opposite direction, the pulsed laser light 14 is not always emitted at predetermined time intervals. Only the internal tissue 13 in a region to be treated is illuminated. Here, a vertical scan period and a horizontal scan period of the pulsed laser light 14 may be determined based on NTSC (National Television Systems Committee) standard. However, it is not necessary that the periods are based on the standard. The periods may be determined in an appropriate manner.

Next, a process of setting a region to be treated will be explained in detail. In this example, the controller 25, the operation unit 29 and the monitor 30, which are illustrated in Figure 1, are configured by a computer system, such as a general personal computer. First, information representing a region-to-be-treated setting pitch is input to the controller 25 through an input means, such as a keyboard or a mouse (not illustrated), which is included in the computer system. The region-to-be-treated setting pitch is an interval between regions to be treated with respect to a predetermined direction of a three-dimensional tomographic image when the regions to be treated are set. In the present embodiment, the predetermined direction is an x-direction, for example.

When the controller 25 receives the region-to-be-treated setting pitch, the controller 25 displays a plurality of two-dimensional tomographic images which are aligned in the x direction at a pitch represented by the region-to-be-treated setting pitch on the monitor 30. Specifically, in this case, a plurality of tomographic images in y-z planes is displayed each pitch on the monitor 30. The plurality of tomographic images is displayed in the lower row of the display screen of the monitor 30, as illustrated in Figure 2. Then, a two-dimensional region to be treated is set in each of the plurality of tomographic images. The two-dimensional region is a region extending both in a vertical direction and in a horizontal direction, and the region is specified by moving a cursor by operating an input means, such as a mouse. It is needless to say that a region to be treated is not set in a tomographic image in which a region to be treated is not recognized. Consequently, the region to be treated is determined as three-dimensional regional information. The three-dimensional regional information is temporarily stored in an internal memory of the controller 25 or the like, for example.

Meanwhile, for example, in treatment of cancers, it is important to know a region which cancer cells have actually reached (degree of infiltration). Therefore, when the plurality of tomographic images is displayed, as described above, it is preferable that two-dimensional images (images in the vertical direction and in the horizontal direction) which represent the state of the region in the depth direction are displayed. In the present embodiment, such two-dimensional images are tomographic images in y-z planes, as described above, or tomographic images in z-x planes.

Next, an actual method for illuminating the three-dimensional region to be treated, which has been set as described above, with the pulsed laser light 14 will be explained. First, the controller 25 reads out the three-dimensional regional information from the internal memory and maps the information onto three-dimensional voxel data to obtain mapping information. Then, the controller 25 extracts a laser light illumination position from the mapping information.

Then, while the region 11 is two-dimensionally scanned with the pulsed laser light 14 in a treatment mode, the controller 25 judges whether the position of an optical axis corresponds to the laser light illumination position, which has been extracted as describe above. (The position of the optical axis is a position at which the center of the beam of the pulsed laser light 14 will pass if the pulsed laser light 14 is emitted.) If the controller 25 judges that the position of the optical axis corresponds to the laser light illumination position, the controller 25 sends a trigger signal to the fs laser 10 to cause the fs laser 10 to emit pulsed laser light 14. If the controller 25 judges that the position of the optical axis does not correspond to the laser light illumination position, the controller 25 does not send a trigger signal. Accordingly, only the internal tissue 13 in the region to be treated is illuminated with the pulsed laser light 14, as illustrated by black dots in Figure 3B.

When two-dimensional scanning on one of x-y planes, illustrated in Figure 2, ends while illumination and non-illumination of the laser light 14 is controlled, the controller 25 inputs a focus control signal S5 to the drive unit 16b of the condensing lens 16. Accordingly, the focal length of the fluid lens 16a increases or decreases by a predetermined value. Consequently, the beam waist position of the pulsed laser light 14 moves by the predetermined value in the depth direction of the internal tissue 13.

In each time when the beam waist position of the pulsed laser light 14 is changed, the processing, as described above, is repeated. Accordingly, only a predetermined region to be treated, which is set in a three-dimensional region of the internal tissue 13, is illuminated with the pulsed laser light 14. Therefore, it is possible to three-dimensionally vaporize or remove the cancer tissue or the like.

Further, in the present embodiment, the means for switching the intensity of the pulsed laser light 14, with which the internal tissue 13 is illuminated, is configured by the controller 25, which controls the output from the fs laser 10. However, the means for switching the intensity of the pulsed laser light 14 may be provided in a different manner, for example, as a separate element. In Figure 4, a turret 50, which is an example of the means for switching the intensity of light, is illustrated. In the turret 50, ND (neutral density) filters 51 and openings 52 are arranged in the circumferential direction of the circular turret 50. The turret 50 is rotated in the direction of the arrow, by a drive means, which is not illustrated. The turret 50 is rotated in synchronization with switching between a diagnosis mode and a treatment mode. When diagnosis is performed, one of the ND filters 51 is inserted to be positioned in the optical path of the pulsed laser light 14, emitted from the fs laser 10. When treatment is performed, one of the openings 52 is positioned in the optical path of the pulsed laser light 14.

When the means for switching the intensity of light is structured, as described above, if the ND filter 51 is inserted in the optical path of the pulsed laser light 14, the pulsed laser light 14 is attenuated by the ND filter. Therefore, the value of the intensity of the pulsed laser light 14, with which a region of a living body is illuminated through the optical fiber 20, is relatively low. In contrast, if the turret 50 is set so that the opening 52 is positioned in the optical path of the pulsed laser light 14, the ND filter 51 is not positioned in the optical path of the pulsed laser light 14. Therefore, the value of the intensity of the pulsed laser light 14, with which the region of the living body is illuminated, is relatively high.

## Claims

1. An optical diagnosis and treatment apparatus comprising:
a pulsed light source (10) for emitting pulsed light (14);
a guide tube (12) which is inserted into a living body;
an illumination optical system (15) for illuminating a region (11) of the living body with the pulsed light (14) through the guide tube (12);
a light condensing means (16) for condensing the pulsed light (14) emitted from the illumination optical system (15);
an optical scan means (17) for two-dimensionally scanning the region (11) of the living body with the condensed pulsed light (14) ;
a light detection means (24) for detecting the pulsed light (14) reflected from the region (11);
an operation means (29) for reconstructing, based on an output from the light detection means (24), a tomographic image of the region (11) which has been illuminated with the pulsed light (14); and
an image display means (30) for displaying the tomographic image based on an output from the operation means (29),
**characterized in that** the apparatus further comprises a light intensity switching means (25, 51) for switching the intensity of the pulsed light (14), with which the region (11) is illuminated, at least between two levels, wherein the two levels are a level at which vaporization of living body tissue due to multi-photon absorption occurs at a convergence position of the pulsed light (14) by the light condensing means (16) and a level at which vaporization does not occur.

2. An optical diagnosis and treatment apparatus, as defined in Claim 1, **characterized in that** the pulsed light source (10) is a femtosecond laser (10).

3. An optical diagnosis and treatment apparatus, as defined in Claim 1 or 2, **characterized in that** the light intensity switching means (25) is a means for changing an output from the pulsed light source (10).

4. An optical diagnosis and treatment apparatus, as defined in Claim 1 or 2, **characterized in that** the light intensity switching means (25, 51) is an ND (Neutral Density) filter (51), which is insertable into and removable from the optical path of the pulsed light (14) emitted from the pulsed light source (10), and which attenuates the pulsed light (14) when the light intensity switching means (51) is inserted into the optical path.

5. An optical diagnosis and treatment apparatus, as defined in any one of Claims 1 to 4, **characterized in that** the light condensing means (16) includes a variable focus mechanism (16b) which can change the convergence position of the pulsed light (14).

6. An optical diagnosis and treatment apparatus as defined in any one of Claims 1 to 5, **characterized in that** the apparatus further comprises a control means (25) for setting the convergence position of the pulsed light (14) with respect to the direction of the two-dimensional scanning and/or the depth direction of illumination thereof based on position information about the reconstructed tomographic image when the intensity of the pulsed light (14) is set at the level at which the vaporization occurs.
